# EUROPEAN PATENT APPLICATION

(11) **EP 4 574 190 A1**
(43) Date of publication of application: **25.06.2025**
(21) Application number: 23854834.1
(22) Date of filing: 08.08.2023
(51) Int. Cl.: A61M 13/00, A61M 11/00

(54) **ADMINISTRATION DEVICE**

(30) Priority: 17.08.2022 JP 2022130224
(71) Applicant: Shin Nippon Biomedical Laboratories, Ltd., Kagoshima 891-1394 (JP)
(72) Inventor: HARUTA, Shunji, Kagoshima-shi, Kagoshima 891-1394 (JP); MISHIMA, Hideaki, Kagoshima-shi, Kagoshima 891-1394 (JP)
(74) Representative: Zacco Sweden AB
(86) International application number: PCT/JP2023/028899
(87) International publication number: WO 2024/038800

(57) **Abstract**

The present invention provides an administration device having a novel structure which is capable of evenly spraying, and thereby stably administering, an object to be administered, such as a powdered medicine, irrespective of who uses such device. The present device is a single-use type administration device 10 for administering a predetermined dose of an object to be administered, such as a powdered medicine, into a nasal cavity, etc. The administration device 10 comprises: a pump member 20 having a tapered shape as a whole, the pump member 20 being deformed when pressed so as to reduce its internal volume; and a cylindrical guide member 30 that is arranged on an inner side of the pump member 20, the guide member 30 guiding a portion that is to be deformed in the pump member. The pump member 20 has a shape that includes a tapered large diameter portion 21 and a tapered small diameter portion 22 which has a diameter smaller than the large diameter portion 21. A stepped portion 23 in which the degree of tapering changes at a midpoint of the tapered shape may be formed between the large diameter portion and the small diameter portion.

## Description

### Technical Field

The present invention relates to an administration device, and more specifically to a structure, etc. of a single use-type administration device for administering a predetermined dose of an object to be administered, such as a powdered medicine, to a nasal cavity, etc.

### Background Art

In general, there has been a known treatment method for dispensing powdered medicine into a nasal cavity of a patient with diseases such as nasal inflammation and nasal allergies. In this treatment method, a dedicated medicine dispensing device is used to dispense a powdered medicine filled in a capsule into a nasal cavity. In addition, a medicine dispensing device used for such treatment method has conventionally been devised (see, for example, Patent Documents 1 and 2).

Operations performed when dispensing a medicine using such medicine dispensing device generally include operations as set forth below. Specifically, a user removes a cap from a cylindrical member and inserts a tip portion into one of his/her nasal cavities, presses a pump so as to allow the air from the pump to flow through an air introduction path into a container, such as a capsule, and this thereby delivers and dispenses medicine in the container into the nasal cavity of the user. The pump used for such medicine dispensing device is typically formed by blow molding which employs a relatively inexpensive mold and is suitable for manufacturing hollow molded products.

### Citation List

### Patent Literature

Patent Literature 1: JPH09-253208 A
Patent Literature 2: JP2004-510559 A

### Summary of Invention

### Technical Problem

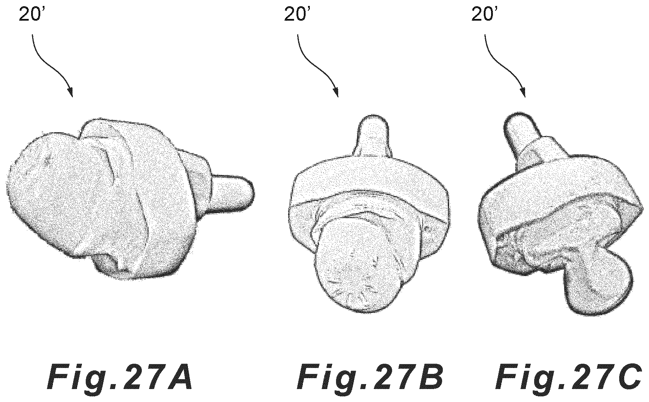

However, the conventional pumps which are manufactured by blow molding as described above greatly vary among products due to poor molding accuracy, and such pumps cannot be collapsed in a uniform way when pressed and may be distorted depending on the product or how it is pressed (see Figure 27). One of the performances required for a medicine dispensing device for nasal administration is having the capability to evenly spray a powdered medicine so as to stably dispense such medicine, irrespective of who uses such device. In light of such point, the feature of the pumps not being able to be collapsed in a uniform way when pressed is simply disadvantageous. In this way, the conventional medicine dispensing device may have a problem in which the way in which the medicine is sprayed varies depending on the operation during administration; however, the fact is that no proposals which achieve improvements from such viewpoint have been provided.

Under the circumstances described above, an object of the present invention is to provide an administration device having a novel structure, which is capable of evenly spraying, and thereby stably administering an object to be administered, such as a powdered medicine, irrespective of who uses such device.

### Solution to Problem

In order to achieve the object above, the inventors have conducted intensive studies. In the first place, considering the background related to the reason why the conventional pumps have been manufactured by blow molding that has poor molding accuracy, it has been found that the use of the blow molding results from the circumstances in which it has been necessary to respond to a demand for reducing the cost of molds, but also the circumstances in which basically no methods other than the blow molding can be employed to mold a pump having a hollow shape with a bellows structure, etc., and having a mouth portion with a reduced diameter, and to remove such pump from the mold. That is to say, in light of various conditions, it has been a general and common-sense way to use a pump having a bellows structure, etc. which is manufactured by blow molding as a pump for a single use-type device for administering an object to be administered, such as a powdered medicine. Considering those circumstances, the inventors have conduced intensive studies about the use of methods other than blow molding and about any new problems which may arise when such other methods are used, and have thereby achieved new findings.

An aspect of the present invention, which has been conceived of based on the above-described findings, provides a single-use type administration device for administering a predetermined dose of an object to be administered, such as a powdered medicine, into a nasal cavity, etc., the device comprising: a pump member having a tapered shape as a whole, the pump member being deformed when pressed so as to reduce its internal volume; and a cylindrical guide member that is arranged on an inner side of the pump member, the guide member guiding a portion of the pump member that is to be deformed.

One of the potential techniques that could replace the poor molding accuracy blow molding is injection molding. It is possible to use injection molding, that has far better molding accuracy than blow molding, to manufacture pumps which have few variations in thickness, etc., and few variations among products; however, in the first place, a pump having a bellows structure, etc. cannot be removed from an injection mold and is therefore unable to be manufactured by injection molding. In other words, in order to manufacture a pump by using injection molding, the pump should have a structure that can allow itself to be removed from the mold, such as a structure in which the pump has a gradually tapered shape as a whole. However, if a pump has such gradually tapered shape as a whole, the pump cannot always be collapsed straight when pressed, and the pump may be tilted to one side, or folded or bent at a midpoint thereof, and it is therefore difficult to achieve a uniform collapsing of the pump. In this regard, in the administration device according to the above-description, since the portion that is to be deformed in the pump member is guided by the guide member, the pump member is not tilted to one side, and is not folded or bent at a midpoint thereof, during the deformation process. That is to say, according to the administration device of the present aspect which employs the pump member that can be manufactured by using injection molding, which has excellent molding accuracy, and that has a structure in which the portion to be deformed is guided by the guide member, it is possible to easily allow the pump to be collapsed in a uniform way when pressed and is therefore possible to evenly spray the powdered medicine and thereby stably dispense the medicine, irrespective of who uses such device.

In the administration device described above, the pump member may be provided with a stepped portion at which the degree of tapering changes at a midpoint of the tapered shape.

In the administration device described above, the pump member may have a shape that includes a tapered large diameter portion and a tapered small diameter portion which has a diameter smaller than the large diameter portion, and the stepped portion may be formed between the large diameter portion and the small diameter portion.

In the administration device described above, the pump member may have a circular horizontal cross-sectional shape.

In the administration device described above, a pressed portion which is to be pressed by a user may be formed at an extremity portion of the pump member where an outer diameter reaches its minimum.

In the administration device described above, the pump member may have a shape that can be molded by injection molding.

In the administration device described above, the cylindrical guide member may be arranged such that at least a portion thereof overlaps with the small diameter portion of the pump member.

In the administration device described above, an outer peripheral surface of the portion of the cylindrical guide member which overlaps with the small diameter portion of the pump member may come into contact with an inner peripheral surface of the small diameter portion.

In the administration device described above, a gap of 3 mm or less may be formed between an outer peripheral surface of the portion of the cylindrical guide member which overlaps with the small diameter portion of the pump member and an inner peripheral surface of the small diameter portion.

In the administration device described above, the cylindrical guide member may have a structure in which: a base end-side cylindrical portion is provided at a base end portion located at an end in a direction along which the pump member is pressed; and an extremity-side cylindrical portion is provided at an extremity portion that is located at an end portion that faces the small diameter portion of the pump member, the extremity-side cylindrical portion extending continuously from the base end-side cylindrical portion.

In the administration device described above, an outer diameter of the base end-side cylindrical portion may be larger than an outer diameter of the extremity-side cylindrical portion.

In the administration device described above, in the cylindrical guide member, an outer peripheral surface of a base end portion located at an end in a direction along which the pump member is pressed is in contact with and fixed to an inner peripheral surface of the large diameter portion of the pump member.

In the administration device described above, a plurality of base end-side slits may be provided at the base end portion of the cylindrical guide member, the base end-side slits being located along a circumferential direction.

In the administration device described above, a sum of widths of the base end-side slits along the circumferential direction of the cylindrical guide member may be 50% or less of an entire circumferential length of the base end portion of the cylindrical guide member.

In the administration device described above, a rod-like guide member may be provided on an inner circumferential side of the extremity portion of the pump member, the rod-like guide member suppressing a sideward movement of the extremity portion when the pump member is deformed.

In the administration device described above, a guide hole may be provided at a central portion of the cylindrical guide member, the guide hole guiding the rod-like guide member so as to be slidable in a longitudinal direction thereof.

In the administration device described above, the rod-like guide member and the guide hole may be provided along a central axis of the administration device.

In the administration device described above, the guide hole may be formed on an inner side of a base end portion of the cylindrical guide member, the base end portion being located at an end in a direction along which the pump member is pressed.

In the administration device described above, a plurality of extremity-side slits may be provided in at least part of the extremity-side cylindrical portion of the cylindrical guide member, the extremity-side slits being located along a circumferential direction.

In the administration device described above, a sum of widths of the extremity-side slits provided in at least part of the extremity-side cylindrical portion may be 75% or less of an entire circumferential length of the extremity-side cylindrical portion.

In the administration device described above, the rod-like guide member may comprise a deformation suppressing portion that extends from a shaft thereof in a radial direction perpendicular to the shaft, the deformation suppressing portion suppressing a sideward movement of the pump member when being deformed.

In the administration device described above, the deformation suppressing portion may be formed in a size and a shape in which at least part thereof comes into contact with an inner peripheral surface of the extremity-side cylindrical portion.

In the administration device described above, the deformation suppressing portion may be formed in a shape in which the deformation suppressing portion extends in a wing-like shape from a shaft thereof in a radial direction perpendicular to the shaft.

In the administration device described above, a tip end portion at a leading end of the rod-like guide member in a sliding direction may have a planar shape, a projected shape, a recessed shape or a tapered shape.

In the administration device described above may further comprise: a nozzle member having a filling space that is filled with the object to be administered and a spray opening through which the object to be administered is sprayed; and a sealing member or a plug member that seals an opening of the filling space, wherein the tip end portion at the leading end of the rod-like guide member may be formed as a puncturing portion that punctures the sealing member or a plunger portion that pushes the plug member upward so as to provide an opening.

In the administration device described above, the pump member may be connected to the opening of the filling space of the nozzle member, and air is sent out in response to a contracting motion of the pump member so as to cause the object to be administered to be sprayed through the spray opening of the nozzle member.

### Advantageous Effect of Invention

According to the present invention, it is possible to provide an administration device having a novel structure, which is capable of evenly spraying, and thereby stably administering, an object to be administered, such as a powdered medicine, irrespective of who uses such device.

### Brief Description of Drawings

[Figure 1] Figure 1 is a perspective view showing an external appearance of a powdered medicine administration device according to an embodiment of the present invention.
[Figure 2] Figure 2 is a perspective view showing an external appearance of the powdered medicine administration device with a tab folded and removed in use.
[Figure 3] Figure 3 is a perspective view showing an external appearance of the powdered medicine administration device with a powdered medicine sprayed therefrom.
[Figure 4] Figure 4 is an exploded perspective view showing the powdered medicine administration device.
[Figures 5A-5E] Figure 5A is a perspective view showing an example of a pump member, and Figures 5B-5E are perspective views showing other examples of pump members.
[Figure 6] Figure 6 is a side view illustrating characteristics of the shape of the pump member.
[Figures 7A and 7B] Figure 7A is a perspective view showing an example of a cylindrical guide member, and Figure 7B is a perspective view showing the pump member with the cylindrical guide member arranged thereinside.
[Figures 8A and 8B] Figure 8A is a vertical cross-sectional view showing an internal structure of the powdered medicine administration device, and Figure 8B is an enlarged view showing an area around a narrowed portion (stepped portion) of the pump member.
[Figures 9A and 9B] Figure 9A is a vertical cross-sectional view showing an internal structure of the powdered medicine administration device immediately after the pump member is pressed, and Figure 9B is an enlarged view showing an area around the narrowed portion (stepped portion) of the pump member.
[Figures 10A and 10B] Figure 10A is a vertical cross-sectional view showing an internal structure of the powdered medicine administration device with the pump member being further pressed, and Figure 10B is an enlarged view showing an area around the narrowed portion (stepped portion) of the pump member.
[Figure 11] Figure 11 is a view illustrating a collapsing margin, etc. of the pump member.
[Figure 12] Figure 12 is a perspective view showing an example of a rod component (rod-like guide member) provided with a pointed piercer portion (puncturing portion) and a wing-like portion (deformation suppressing portion).
[Figures 13A-13C] Figures 13A to 13C are vertical cross-sectional views each showing the powdered medicine administration device having the rod component (rod-like guide member) provided with the wing-like portion (deformation suppressing portion), in which Figure 13A shows a state before the pump member is pressed, Figure 13B shows a state immediately after the pump member is pressed, and Figure 13C shows a state where the pump member is further pressed.
[Figures 14A and 14B] Figures 14A and 14B are vertical cross-sectional views each showing an internal structure of the powdered medicine administration device, in which Figure 14A shows the case where the rod component (rod-like guide member) has the wing-like portion (deformation suppressing portion), and Figure 14B shows the case where the rod component does not have the wing-like portion.
[Figures 15A-15E] Figures 15A-15E are views each showing an example of the shape of the rod component (rod-like guide member) provided with the pointed piercer portion (puncturing portion).
[Figures 16A and 16B] Figures 16A and 16B are diagrams each illustrating the motion and effect of the powdered medicine administration device in the case where it is provided with the rod component (rod-like guide member), in which Figure 16A is a vertical cross-sectional view showing an internal structure of the powdered medicine administration device before the pump member is pressed, and Figure 16B is an enlarged view showing an area around the pointed piercer portion.
[Figures 17A and 17B] Figures 17A and 17B are diagrams each illustrating the motion and effect of the powdered medicine administration device in the case where it is provided with the rod component (rod-like guide member), in which Figure 17A is a vertical cross-sectional view showing an internal structure of the powdered medicine administration device immediately after the pump member is pressed, and Figure 17B is an enlarged view showing an area around the pointed piercer portion.
[Figures 18A and 18B] Figures 18A and 18B are diagrams each illustrating the motion and effect of the powdered medicine administration device in the case where it is provided with the rod component (rod-like guide member), in which Figure 18A is a vertical cross-sectional view showing an internal structure of the powdered medicine administration device with the pump member being further pressed, and
Figure 18B is an enlarged view showing an area around the pointed piercer portion.
[Figures 19A and 19B] Figures 19A and 19B are views showing, in the order from Figure 19A to Figure 19B, the outline of the motion of the rod component (rod-like guide member) provided with the pointed piercer portion (puncturing portion) when the pump member is pressed.
[Figures 20A-20C] Figures 20A and 20B are views showing, in the order from Figure 20A to Figure 20B, the outline of how a plug member is pushed out by a tip end portion at a leading end of the rod component (rod-like guide member) having a planar shape when the pump member is pressed, and Figure 20C is a view showing an example in which the plug member has a different shape.
[Figures 21A-21C] Figure 21A is a perspective view showing the cylindrical guide member, Figure 21B is a perspective view showing the rod component (rod-like guide member) with a planar-shape tip end portion at the leading end, and Figure 21C is a perspective view showing a state in which the cylindrical guide member and the rod component are combined.
[Figure 22] Figure 22 is a side view showing an example of an arrangement of the pump member and the cylindrical guide member.
[Figure 23] Figure 23 is a side view showing an example of the cylindrical guide member.
[Figures 24A-24D] Figure 24A is a perspective view, Figure 24B is a plan view as viewed from direction b, Figure 24C is a perspective view as viewed from below along direction c, and Figure 24D is a bottom view as viewed from direction d, each showing another form example of the cylindrical guide member.
[Figure 25] Figure 25 is a perspective view showing a further form example of the cylindrical guide member.
[Figures 26A and 26B] Figures 26A and 26B are views each showing a form example of the cylindrical guide member provided with extremity-side slits.
[Figures 27A-27C] Figures 27A-27C are views each showing, as reference, an example of a pump member which has not been collapsed as intended in a conventional powdered medicine administration device that is not provided with a cylindrical guide member.

### Description of Embodiments

Now the configuration of the present invention will be described in detail below, based on an example of an embodiment shown in the attached drawings.

### [First Embodiment]

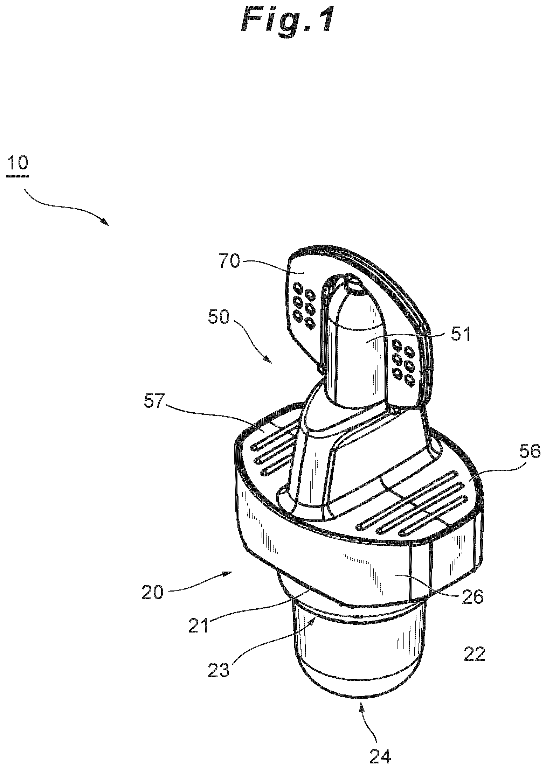

A nasal administration device (hereinafter referred to as the "powdered medicine administration device" or simply as the "administration device") 10 for dispensing and administering a powdered medicine into a nasal cavity is a single use-type (non-reusable type) device which is suitable for dispensing a predetermined dose of powdered medicine into a nasal cavity of a patient. The powdered medicine administration device 10 of the present embodiment includes a pump member 20, a cylindrical guide member 30, a rod component (rod-like guide member) 40, a nozzle member 50, a sealing member 60, a tab (closing member) 70, etc. (see Figures 1-4, etc.).

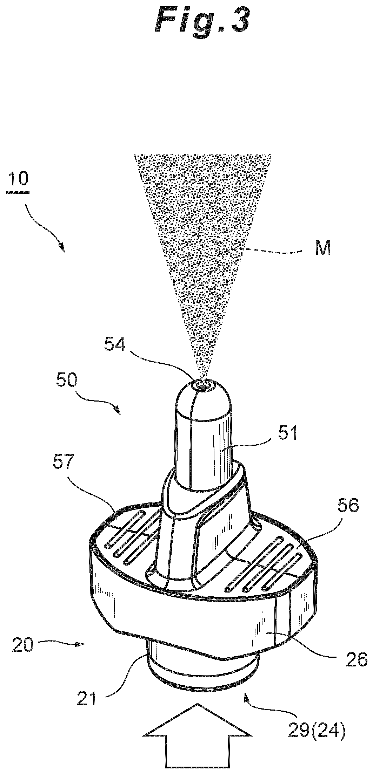

The pump member 20 is a member that sends out air to cause a powdered medicine M, which is an object to be administered, to be sprayed from a spray opening 54 of the nozzle member 50, when the powdered medicine administration device 10 is used. The present embodiment employs, as the pump member 20, a member having a structure in which, when a pressed portion 29 located at a bottom thereof is pressed by a user with his/her finger, the pump member 20 is collapsed and the interior volume thereof is reduced (see Figure 3, etc.). Although the orientation of the powdered medicine administration device 10 during use is not strictly limited, it is normally assumed to be used in an orientation in which the spray opening 54 of the nozzle member 50 faces upward. In the present embodiment, the following description will be provided assuming that the orientation in which the spray opening 54 of the nozzle member 50 faces vertically upward is referred to as a usage orientation, and the side where the spray opening 54 of the nozzle member 50 is located in such orientation is referred to as an "upper" side and the opposite side is referred to as a "lower" side (see Figure 3, etc.), for the purpose of illustration.

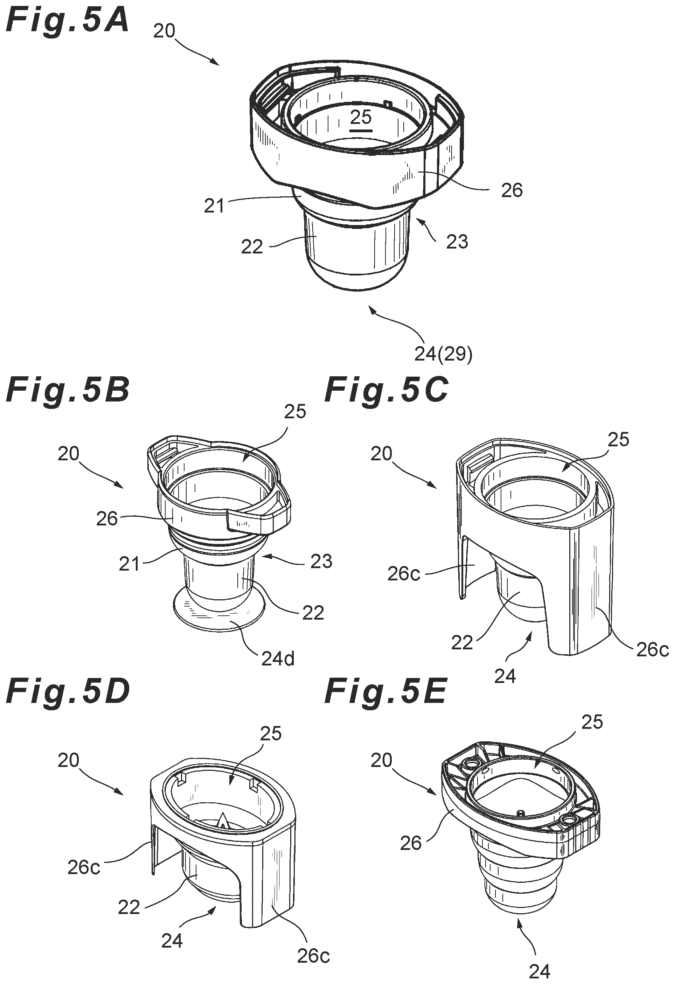

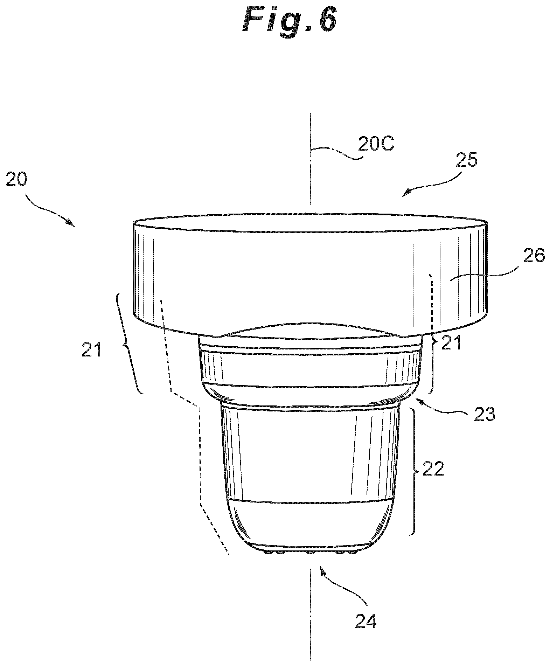

The specific shape of the pump member 20 is not particularly limited, as long as it functions as described above. As an example, the present embodiment employs the pump member 20 having a shape that can be molded by injection molding. Conventionally, many pump members used for such powdered medicine administration device have been manufactured by blow molding that has a poor molding accuracy. However, by employing, in place of blow molding, injection molding which is far superior in molding accuracy as compared to blow molding, it is possible to achieve a pump member with few variations in thickness, etc. and few variations among products. However, if injection molding is employed, the pump member needs to have a shape in which the diameter of an opening 25 is larger than the diameter of the other portion, so that such pump member can be removed from the mold along a central axis 20C after molding. In consideration of various points, the present embodiment employs the pump member 20 having a gradually tapered shape as a whole, with an extremity portion (hereinafter also referred to as the "bottom portion") 24, which is located at a bottom in the usage positioning, being closed, and the opposite side being opened as the opening 25 (see Figures 5A, 6, etc.). The pump member 20 is provided with a large diameter portion 21, a small diameter portion 22, a narrowed portion (stepped portion) 23, the bottom portion 24, and the opening 25.

The large diameter portion 21 is a portion with a large diameter, which is included in a portion that is to be collapsed and deformed in the pump member 20. The large diameter portion 21 of the present embodiment is formed in a tapered shape in which the diameter gradually decreases from the opening 25 toward the bottom portion 24 (see the dashed line in Figure 6, which shows how the large diameter portion 21 is tapered).

The small diameter portion 22 is a portion having a smaller diameter than the large diameter portion 21, which is included in the portion that is to be collapsed and deformed in the pump member 20. The small diameter portion 22 in the present embodiment is formed in a tapered shape in which the diameter gradually decreases toward the bottom portion 24 (see the dashed line in Figure 6, which shows how the small diameter portion 22 is tapered).

The narrowed portion 23 is formed as a portion in which the degree of tapering changes in a middle area of the pump member 20 that has a tapered shape as a whole as described above. For example, in the present embodiment, the narrowed portion 23 in which the degree of tapering is larger (the inclination thereof with respect to the central axis 20 is larger) than those of the large diameter portion 21 and the small diameter portion 22 is formed between the large diameter portion 21 and the small diameter portion 22 (see Figure 6, etc.).

The bottom portion (extremity portion) 24 is a portion located at an extremity of the tapered small diameter portion 22 which is included in the portion that is to be collapsed and deformed in the pump member 20, and the bottom portion 24 may be formed in a tapered shape such as, for example, a dome shape (see, Figure 6, etc.). In the usage orientation of the powdered medicine administration device 10 with the spray opening 54 of the nozzle member 50 facing upward, the bottom portion 24 faces downward and functions as the pressed portion 29 on which the user applies his/her thumb to press during use (see Figure 3, etc.). Although not shown in detail, as a general usage form, when the powdered medicine administration device 10 is actually used, the user hooks his/her index finger and middle finger respectively around finger hook portions 56, 57 which are arranged so as to sandwich the nozzle portion 51, and presses the pressed portion 29 at the bottom portion 24 with his/her thumb, thereby causing the powdered medicine M to be sprayed from the spray opening 54 of the nozzle member 50.

The opening 25 serves as an opening in the large diameter portion 21 which is included in the portion that is to be collapsed and deformed in the pump member 20 (see Figure 6, etc.). Although the portion that is to be collapsed and deformed in the pump member 20, i.e., the tapered portion extending from the opening 25 to the bottom portion 24, is formed in a conical shape having a circular horizontal cross-sectional shape in the present embodiment, such shape is merely a preferred example, and a different shape may be employed.

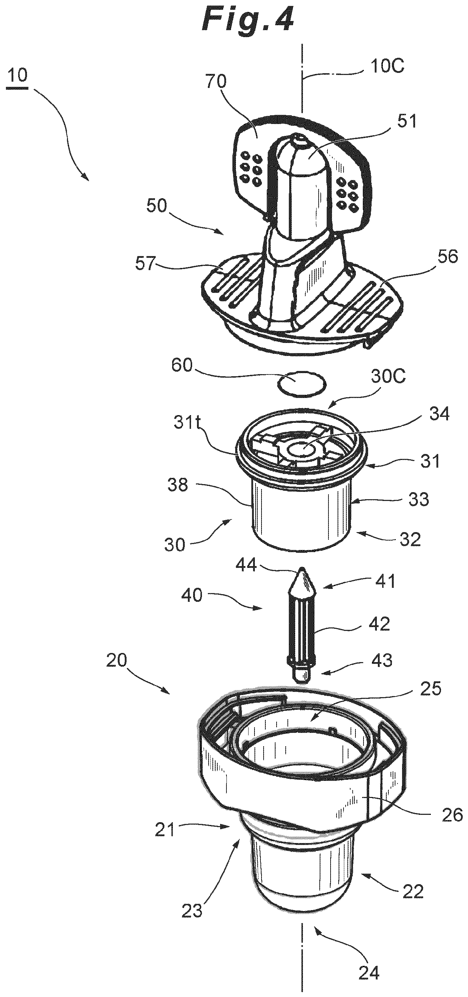

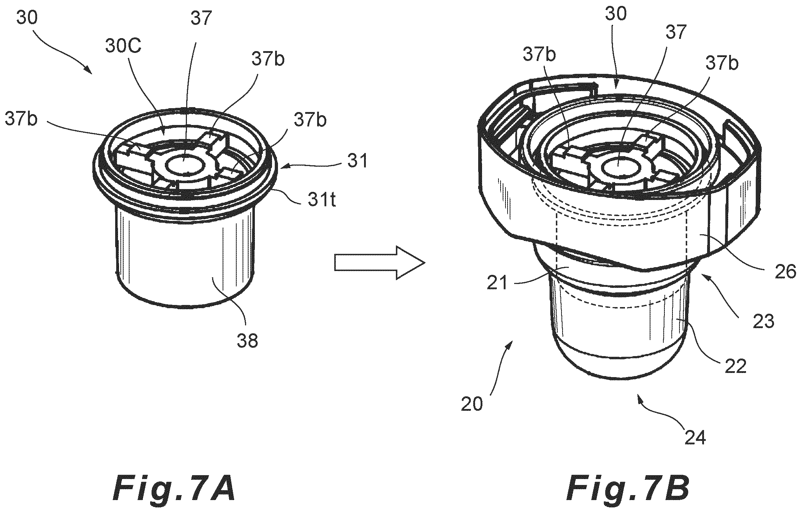

The pump member 20 in the powdered medicine administration device 10 of the present embodiment is further provided with a base portion 26 (see Figures 6, 7, etc.). The base portion 26 has a similar shape (e.g., an elliptical shape) to that of the nozzle member 50 (described later) and is formed so as to be integrated with the nozzle member 50 and thereby constitute a casing of the powdered medicine administration device 10 (see Figures 4, 5(A), etc.). In the present embodiment, the portion that is to be collapsed and deformed (i.e., the large diameter portion 21, the small diameter portion 22 and the narrowed portion 23) in the pump member 20 and the base portion 26 are formed in an integrated manner, however, the pump member 20 may be constituted by separately molding the pump member 20 and the base member 26 and later integrating them by, for example, partially welding them together.

The above-described entire shape and structure of the pump member 20 provided with the base portion 26 is merely a preferred example. The base portion 26 may have another shape so as to fit the shape of the nozzle member 50 (see Figures 5B, 5E, etc.), or may have a shape that further has a pump cover 26c (see Figures 5C and 5D). The pump cover 26c is provided in order to prevent the deformation portion of the pump member 20 from being unintentionally collapsed or to prevent the powdered medicine M from being unintentionally sprayed before use, and to give the user the impression that the powdered medicine administration device 10 can be operated by pressing the extremity portion 24 of the pump member 20 upward, and the pump cover 26c is constituted by a wall member that covers both sides of a periphery including the large diameter portion 21, the small diameter portion 22, etc. (see Figure 5C, etc.). In order to allow for the user's operation of pressing the bottom portion 24 with his/her finger (thumb in most cases), the pump cover 26c is not provided on the front side (or the rear side) of the pump member 20. The bottom portion 24 may be provided with a dish-like portion 24d which increases a contact area with the user's finger (see Figure 5B).

The cylindrical guide member 30 is arranged on an inner side of the pump member 20 described above and provided so as to guide the portion that is to be deformed in the pump member 20. The specific structure of the cylindrical guide member 30 is not particularly limited as long as it has a function of guiding the above-described portion. In one example, a substantially cylindrical member which opens at both ends is arranged on an inner side of the pump member 20 and used as the cylindrical guide member 30 in the present embodiment (see Figure 4, etc.). The cylindrical guide member 30 is integrally formed with the nozzle member 50 by, for example, fitting a base end portion 31 thereof with the nozzle 50.

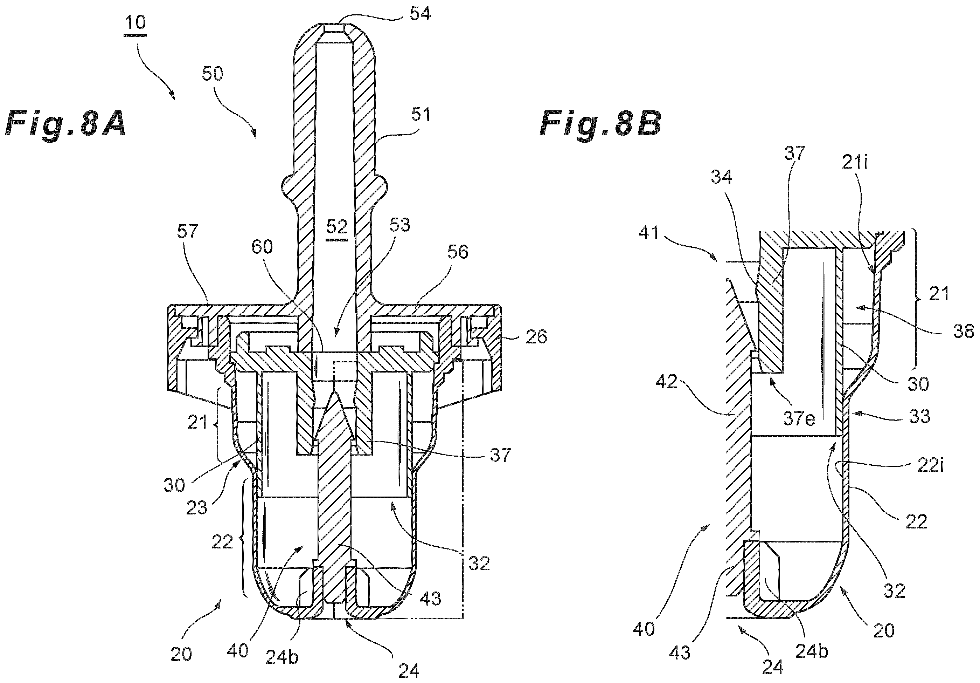

The cylindrical guide member 30 is arranged such that at least a portion thereof overlaps with the small diameter portion 22 of the pump member 20. For example, the cylindrical guide member 30 of the powdered medicine administration device 10 according to the present embodiment is provided such that at least part of a lower end portion 32 thereof overlaps with the small diameter portion 22 of the pump member 20 (see Figures 8, 11, 22, etc.). In this case, the portion of an outer peripheral surface 33 of the cylindrical guide member 30 which overlaps with the small diameter portion 22 of the pump member 20 may come into contact with an inner peripheral surface 22i of the small diameter portion 22; however, such portion does not necessarily have to come into contact with the inner peripheral surface 22i and may alternatively be spaced apart therefrom. If such portion is spaced apart from the inner peripheral surface 22i, it is preferred for the size of a gap between the outer peripheral surface 33 of the cylindrical guide member 30 and the inner peripheral surface 22i of the small diameter portion 22 to be equal to or smaller than a predetermined value (e.g., 3 mm or less), so that the function of the cylindrical guide member 30 of guiding the portion that is to be deformed in the pump member 20 is sufficiently exercised.

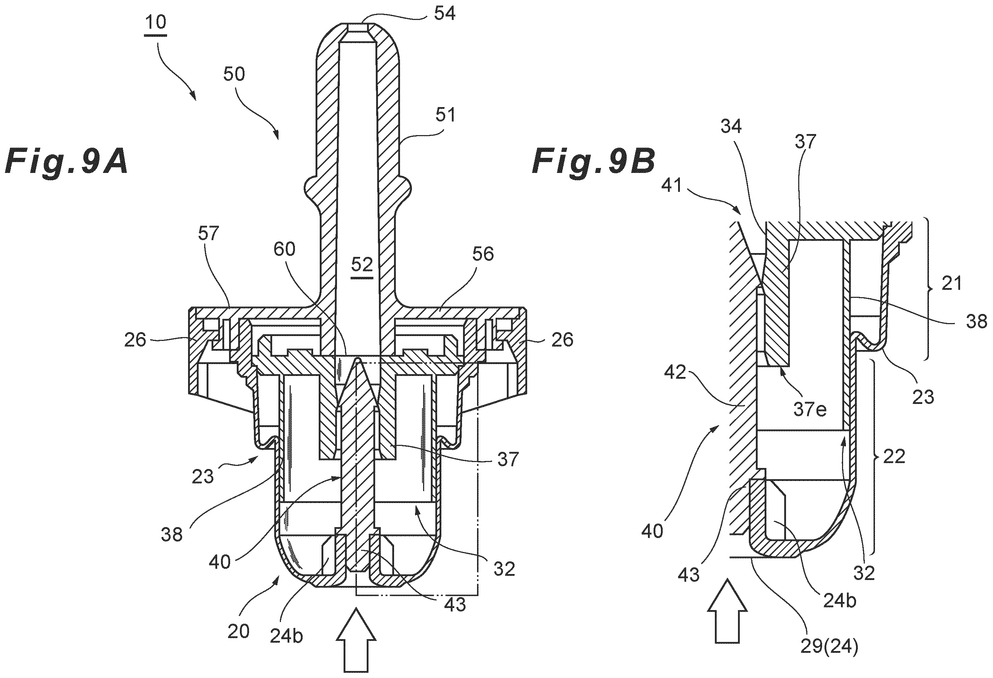

A central portion 30C of the cylindrical guide member 30 is provided with a guide hole 34 that guides the rod component (rod-like guide member) 40 so as to be slidable in the longitudinal direction thereof (see Figures 4, 9, etc.). **The** guide hole 34 in the powdered medicine administration device 10 in the present embodiment is provided so as to extend along the central axis (denoted by reference symbol 10C in Figure 4) of the powdered medicine administration device 10.

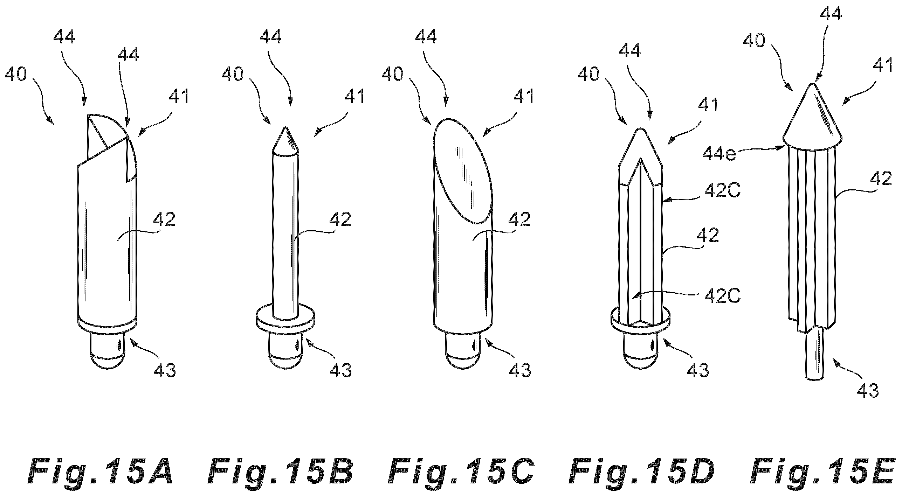

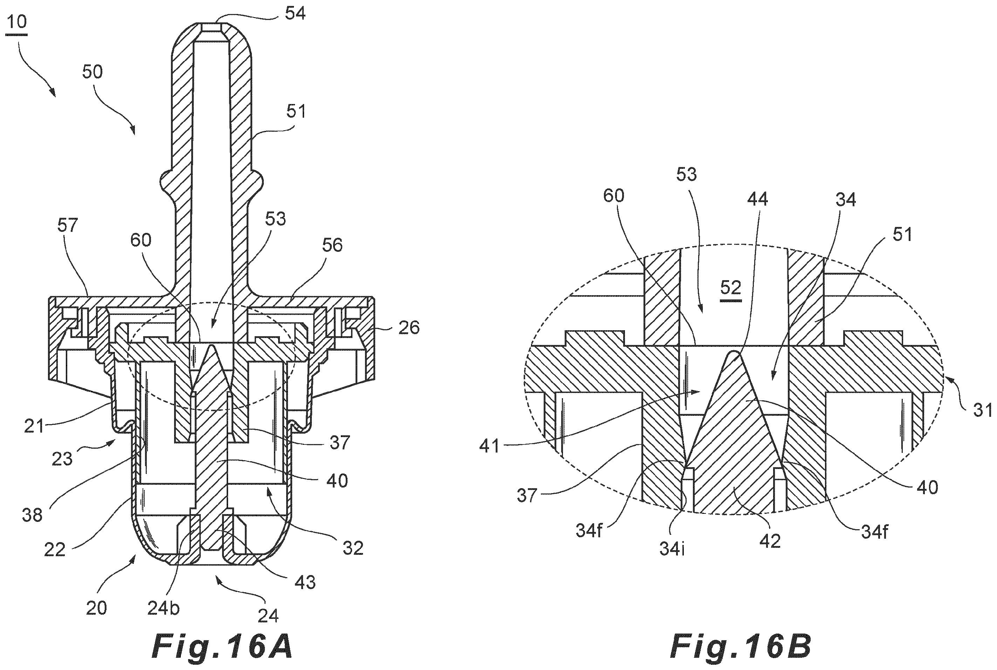

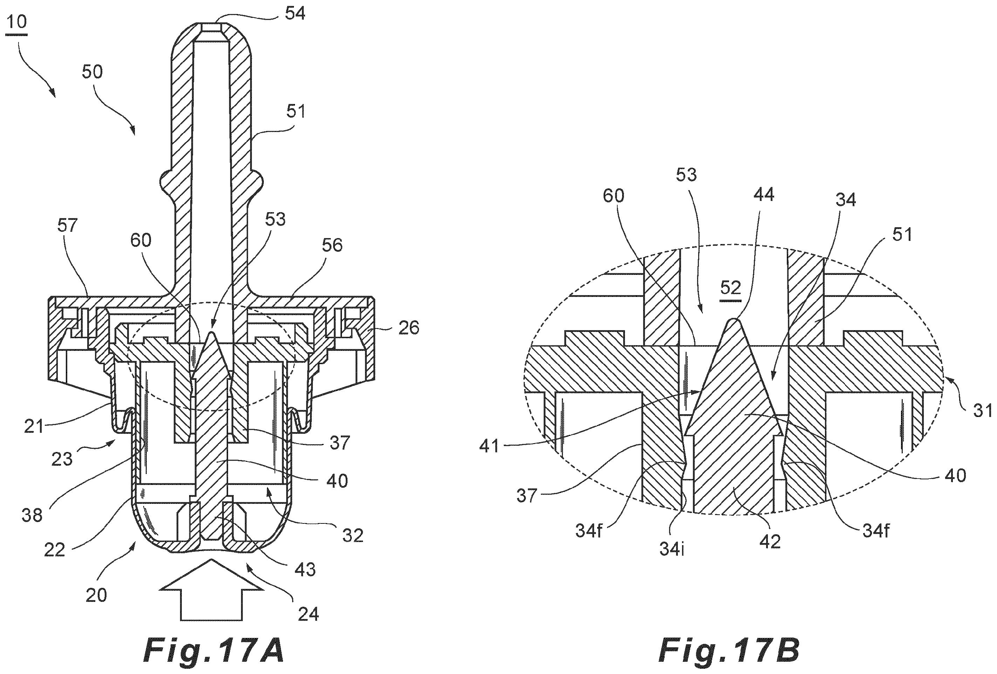

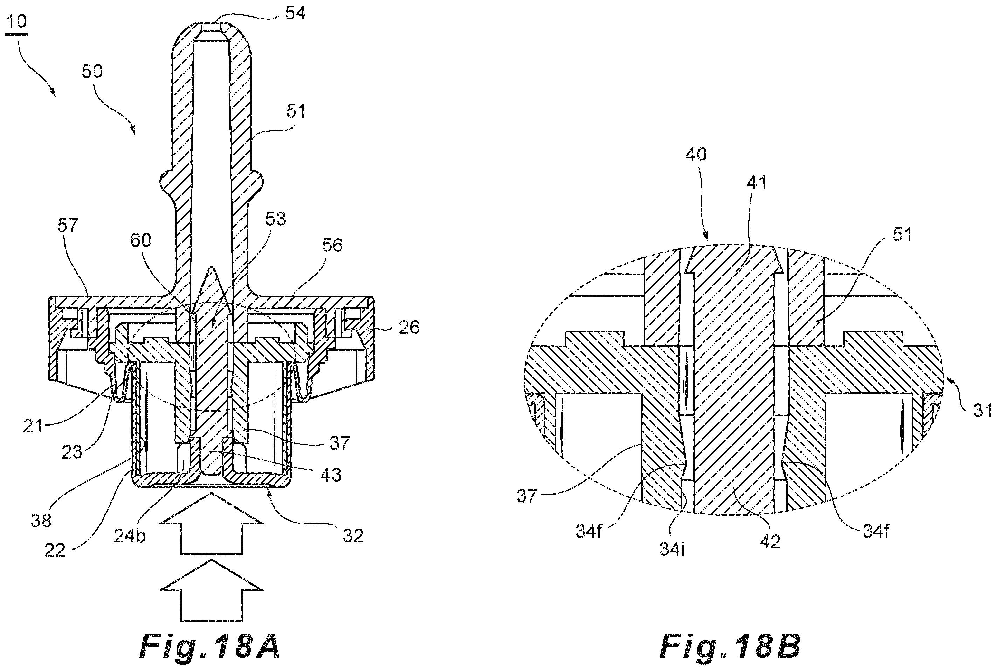

The inner wall 34i of the guide hole 34 is provided with a locking portion 34f for rendering the rod component 40 in a locked state at a predetermined position (see Figure 16, etc.). The locking portion 34f is formed so as to have a diameter that is slightly smaller than the diameter of a rim portion 44e (see Figure 15E, etc.) having the maximum diameter in the pointed piercer portion 44 of the rod component 40 (see Figure 16B, etc.). Before the powdered medicine administration device 10 is used, the rim portion 44e of the pointed piercer portion 44 is locked by the locking portion 34f, whereby the rod component 40 remains at the predetermined position (see Figure 16). When the pump member 20 in such powdered medicine administration device 10 is pressed to thereby cause a force that exceeds a predetermined value to act on the rod component 40, the rim portion 44e of the pointed piecer part 44 moves across the locking portion 34f, whereby the rod component 40 starts moving upward in a manner guided along the guide hole 34 (see Figures 17 and 18).

The rod component 40 is a rod-like guide member provided on the inner peripheral side of the bottom portion 24 of the pump member 20. The rod component 40 includes a shaft 42 that extends linearly, and the rod component 40 moves in the guide hole 34 of the cylindrical guide member 30 along the central axis 10C (see Figures 4, 8, etc.). A lower end portion 43 of the rod component 40 is mounted in the vicinity of the center of the bottom portion 24 of the pump member 20 such that the rod component 40 extends perpendicularly to the bottom portion 24. For example, in the present embodiment, the lower end portion 43 having a smaller diameter than the shaft 42 may be inserted into a boss portion 24b formed on the bottom portion 24 of the pump member 20 to thereby mount the rod component 40 in the pump member 20 (see Figure 8, etc.). In the powdered medicine administration device 10 having such rod component 40, when the user presses and deforms the pump member 20, the bottom portion (extremity portion) 24, the small diameter portion 22, etc. can be prevented from moving sideward, so that they can be pushed straight upward. Considering the fact that the motion of fingers for pressing the pump member 20 varies among individual users and there is usually no reproducibility in fine motions during the pressing operation, the powdered medicine administration device 10 of the present embodiment can achieve a uniform operation that causes the pump member 20 to be pushed straight upward, regardless of who uses such device.

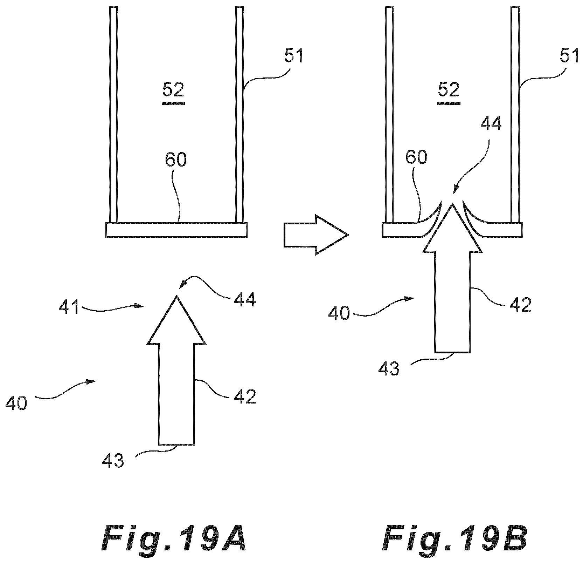

An upper end portion 41 (being a tip end portion at the leading end of the rod component 40 in the sliding direction when the pump member 20 is pressed) of the rod component 40 may have various shapes, such as a planar shape, a projected shape, a recessed shape, or a tapered shape, depending on the intended use. The rod component 40 of the powdered medicine administration device 10 of the present embodiment is formed in a shape wherein the upper end portion 41 thereof is tapered with its end pointed, so as to have a pointed piercer portion (puncturing portion) 44 (see Figure 4, etc.). In this way, the rod component 40 having the pointed piercer portion 44 formed on its end also functions as a piercer, such that the pointed piercer portion 44 of the upper end portion 41 punctures the sealing member 60 when the pump member 20 is pressed (see Figures 9, 13, 16-19, etc.). Although Figure 9 and other drawings show the rod component 40 having a conical upper end portion 41, this is merely a preferred example. The shape of the upper end portion 41 may be a shape having an inclined elliptic surface (see Figure 15C), or a shape having two pointed ends with two inclined semi-elliptic surfaces which are inclined in different directions (see Figure 15A), etc., in addition to the conical shape (see Figures 15B and 15E) or a cone-like shape formed on the shaft 42 having a cross-like cross-sectional shape with grooves 42C formed on four sides (see Figure 15D), as long as the rod component 40 can function as a piercer.

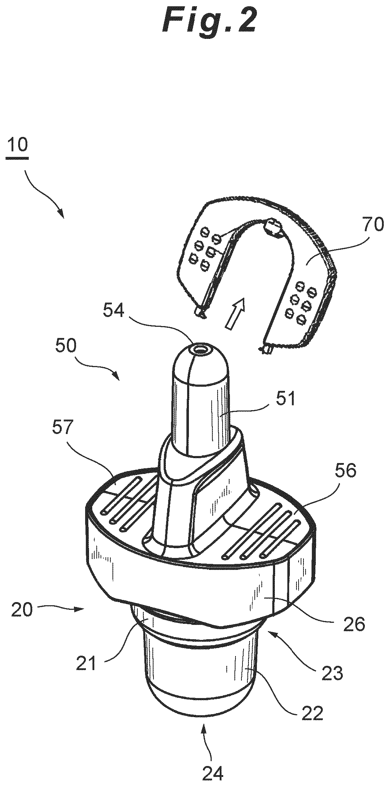

The nozzle member 50 includes a nozzle portion 51 that allows the powdered medicine M to be easily dispensed into a nasal cavity of a patient. The nozzle portion 51 has a tip end which is appropriately rounded as needed (see Figures 4, 8, etc.). A filling space 52 that is filled with the powdered medicine M is formed inside the nozzle member 50 (see Figure 8A, etc.). The spray opening 54 for spraying the powdered medicine M is provided at the center of the tip end of the nozzle member 50 (see Figure 2, etc.). An opening 53 through which the powdered medicine M can be loaded into the filling space 52 is formed on a base end side (i.e., the side to be mounted on the pump member 20) of the nozzle member 50 (see Figure 8, etc.). In addition, the nozzle member 50 is also provided with the finger hook portions 56, 57 which are arranged so as to sandwich the nozzle portion 51 (see Figure 4, etc.).

The sealing member 60 seals the opening 53 of the filling space 52 of the nozzle member 50 (see Figures 3B and 3C). The sealing member 60 may be a moisture proof film (e.g., aluminum sheet) that seals the filling space 52 to prevent the powdered medicine M from being degraded by air or moisture, and the sealing member 60 preferably uses a member that can easily be pierced by the pointed piercer portion 44. The present embodiment employs the sealing member 60 that maintains airtightness or sealing performance without being broken or peeled off even when the internal pressure of the pump member 20 increases to a predetermined level.

The tab 70 closes the spray opening 54 of the nozzle member 50. The tab 70 of the present embodiment is removably attached to the tip end of the nozzle member 50, and when, in use, the user folds and removes the tab 70 with his/her hand, the spray opening 54 is opened (see Figures 1, 2, etc.).

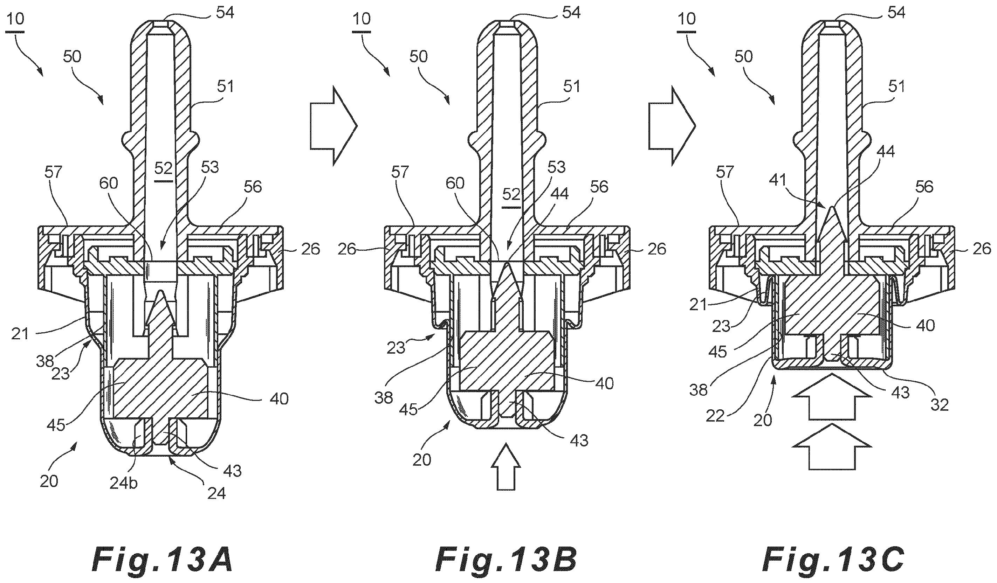

The powdered medicine administration device 10 as described above has a structure in which the pump member 20 is connected to the opening 53 of the filling space 52 of the nozzle member 50 (see Figure 8, etc.), and as a result of the operation of collapsing and contracting the pump member 20, the air is sent out from the pump member 20, which causes the powdered medicine M to be sprayed from the spray opening 54 of the nozzle member 50 (see Figure 13, etc.). The powdered medicine administration device 10 of the present embodiment has the pump member 20 having a shape in which the large diameter portion 21 and the small diameter portion 22 are connected via the narrowed portion 23, and such pump member 20 is easily collapsible when the pressed portion 29 in the extremity portion 24 is pressed. Furthermore, the narrowed portion 23 which is provided between the large diameter portion 21 and the small diameter portion 22 such that the degree of tapering changes functions as a starting point of deformation (the point where deformation starts) when the pump member 20 is collapsed (see Figures 8, 9, etc.). The pump member 20 having such shape can easily be collapsed as intended along the central axis 20C when the pressed portion 29 is pressed. Furthermore, in the powdered medicine administration device 10 of the present embodiment in which the pump member 20 is molded by injection molding that is excellent in molding accuracy, there are few variations in the thickness, etc. of the pump member 20 and little margin of error among products; therefore, the pump member 20 can be made such that it is easily collapsible as intended in every product. In addition, since the powdered medicine administration device 10 of the present embodiment includes the cylindrical guide member 30 that guides the portion that is to be deformed in the pump member 20, the pump member 20 can easily be collapsed in a uniform way, which makes it possible to allow the powdered medicine M to be sprayed evenly and thereby be stably dispensed, irrespective of who uses such device.

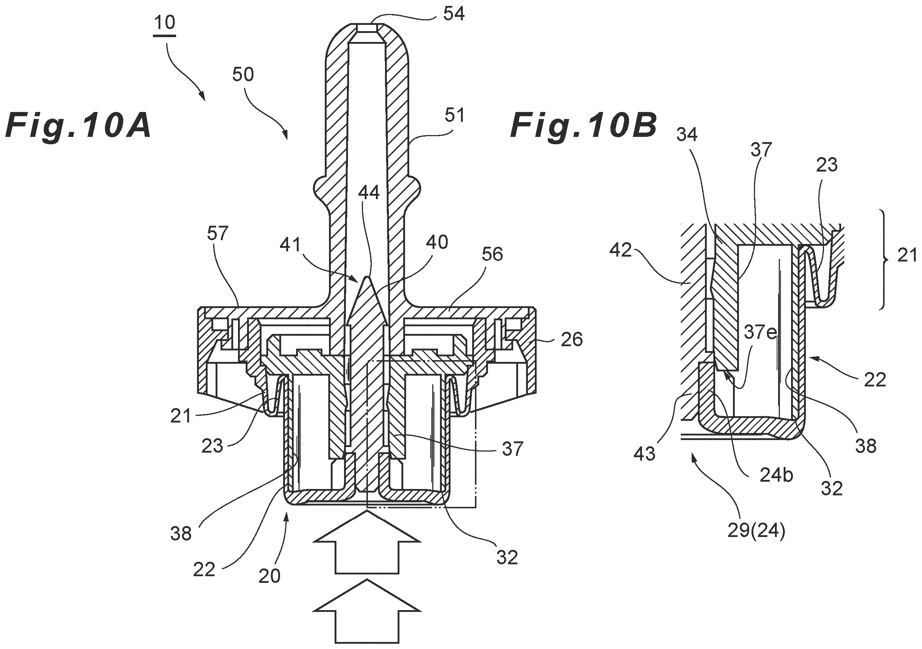

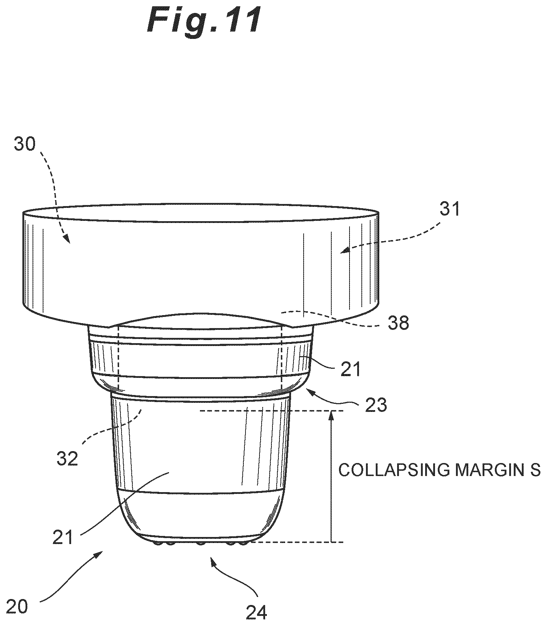

**It** should be noted that the slidable length of the rod component 40 is the length by which the entire rod component 40 is accommodated within the guide hole 34, or the length by which part of the rod component 40 abuts on an end portion 37e of an inner cylindrical portion 37 that constitutes the guide hole 34 (see Figure 10) when the pump member 20 is pressed so as to cause the rod component 40 to slide as described above (see Figures 8, 9, etc.). The collapsing amount of the pump member 20 corresponding to the slidable length of the rod component 40, i.e., the maximum deformation amount along the central axis 10C of the pressed pump member 20, corresponds to the "collapsing margin" S of such pump member 20 (see Figure 11).

### [Second Embodiment]

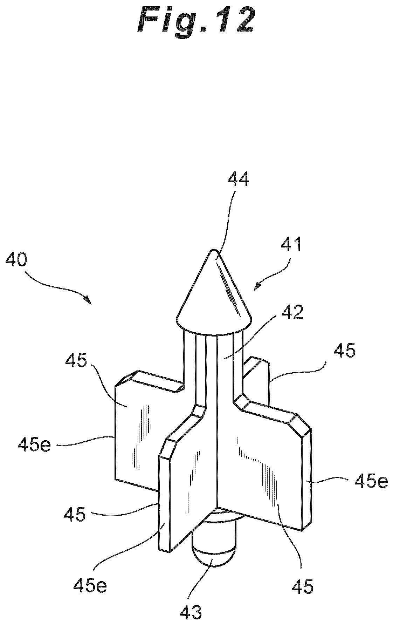

The rod component 40 of the powdered medicine administration device 10 may further include a deformation suppressing portion that suppresses a sideward movement of the rod component and also helps the rod component 40 to be pushed straight upward when the pump member 20 is deformed. The following description will now describe the powdered medicine administration device 10 having such deformation suppressing portion as the second embodiment of the present invention (see Figures 12, 13, etc.). It should be noted that the following description will mainly describe the parts of the configuration which are different from the first embodiment described above.

The rod component 40 of the powdered medicine administration device 10 of the present embodiment includes four wing-like deformation suppressing portions (hereinafter also referred to as the "wing-like portions") 45 that each extend from the shaft 42 in a radial direction (see Figure 12, etc.). The wing-like portions 45 of the present embodiment each have a size and shape in which at least part (e.g., an outer peripheral edge 45e) thereof comes into contact with an inner peripheral surface of an extremity-side cylindrical portion 36 of the cylindrical guide member 30; however, this is merely a preferred example, and the wing-like portion 45 may have a size and a shape in which a predetermined gap is formed between itself and the inner peripheral surface of the extremity-side cylindrical portion 36.

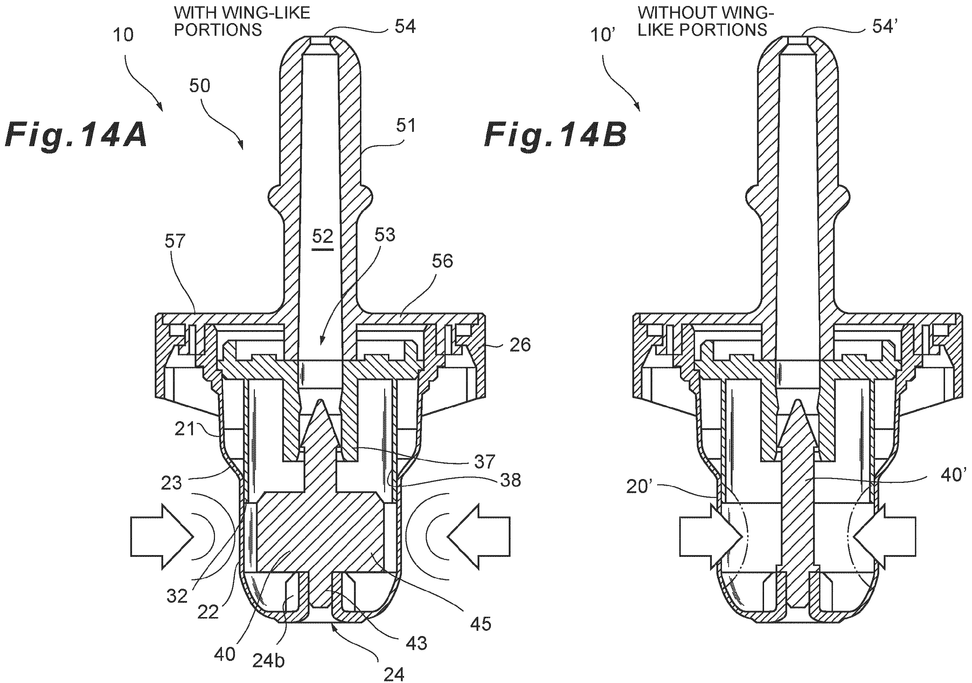

According to the powdered medicine administration device 10 having the rod component 40 that includes the above-described wing-like portions 45, the wing-like portions 45 function as deformation suppression means in the case of an incorrect operation of the pump, which makes it possible to prevent the pump member 20 from being collapsed from its lateral sides and to further help the rod component 40 to be pushed straight upward in conjunction with the other functions (see Figures 13 and 14A). Meanwhile, in a conventional powdered medicine administration device which does not have such wing-like portions, when external force unexpectedly acts thereon, a pump member 20' could be collapsed (see Figure 14(B); it should be noted that, in Figure 14B and Figure 28, reference numerals with " ' " are used for corresponding members to those in the above-described powdered medicine administration device 10).

The shape and size of the wing-like portions 45 are not particularly limited as long as they are capable, as their primary function, of suppressing the pump member 20 from being unintentionally deformed due to an incorrect operation of the pump member 20 and further capable, as needed, of helping the rod component 40 to be pushed straight upward. For example, although the present embodiment shows the wing-like portions 45 that extend from the shaft 42 in the radial direction, this is merely a preferred example (see Figure 12, etc.). In addition to such shape, the wing-like portions 45 may be formed in shapes in which they are curved, or bended at a midpoint, in a plan view (or in a bottom view) as viewed along the central axis 10C.

### [Third Embodiment]

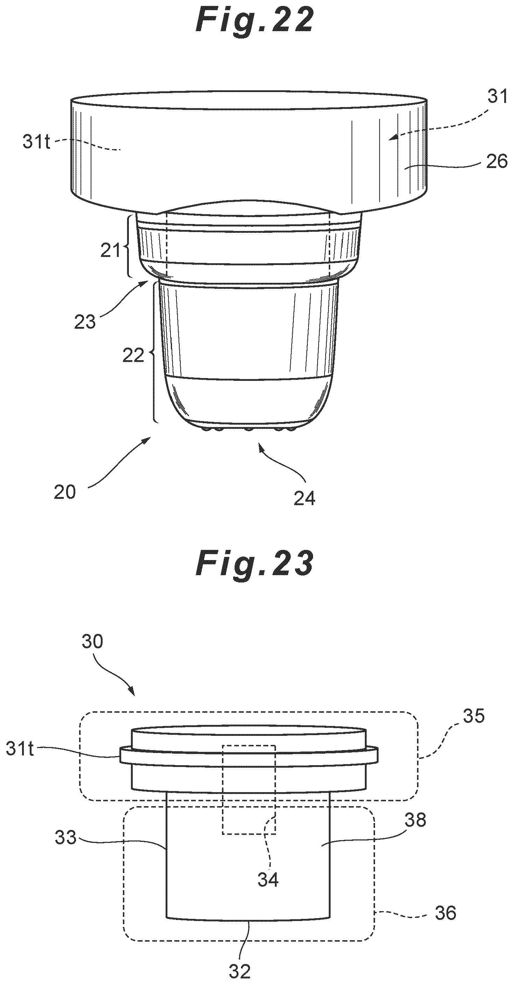

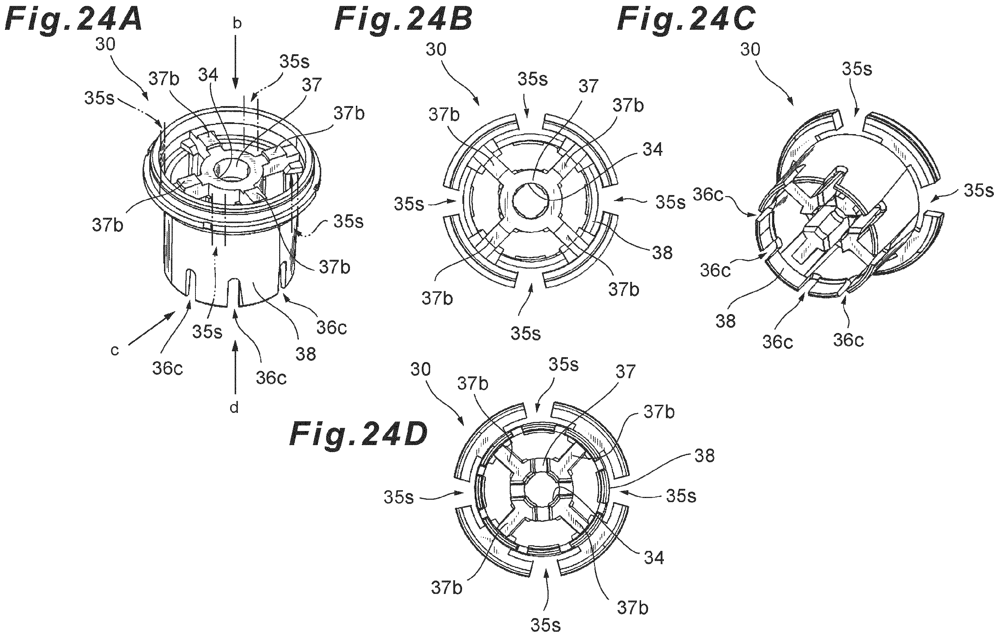

Now, the following description will describe, as the third embodiment of the present invention, a powdered medicine administration device 10 that has an inner cylindrical portion 37 on the inner side of the base end portion 31 of the cylindrical guide member 30 (see Figures 22, 24, etc.). The following description will mainly describe the parts of the configuration which are different from the above-described embodiments.

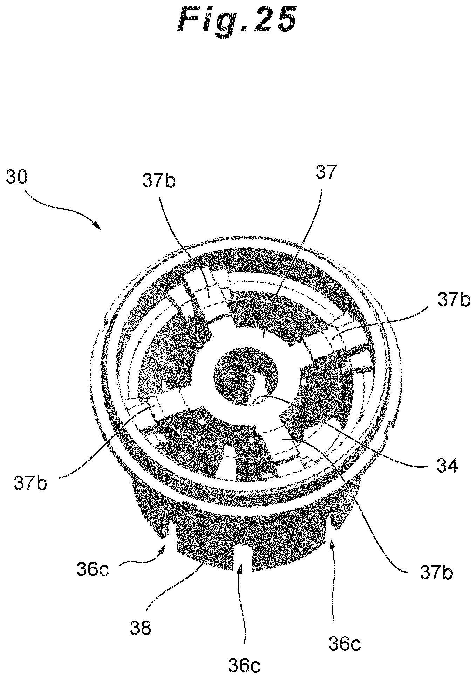

In the powdered medicine administration device 10 of the present embodiment, the inner cylindrical portion 37 that constitutes the guide hole 34 is formed on the inner side of the base end portion 31 (which refers to an end portion located at an end in the direction along which the pump member 20 is pressed, which corresponds to an upper end portion in the powdered medicine administration device 10 of the present embodiment; therefore, the base end portion will be described as the "upper end portion" in the following description) of the cylindrical guide member 30 (see Figure 25, etc.). The inner cylindrical portion 37 is connected, via beams arranged on four sides thereof and extending radially into a cross-like shape, to a base end-side cylindrical portion 35 of the cylindrical guide member 30 or to a portion extending from the base end-side cylindrical portion 35 to at least part of the extremity-side cylindrical portion 36 in the cylindrical guide member 30.

An outer cylindrical portion 38 is arranged such that at least a portion thereof overlaps with an area of the pump member 20 which extends from part of the large diameter portion 21 via the narrowed portion 23 to part of the small diameter portion 22 in a side view (see Figure 22). The outer cylindrical portion 38 of the cylindrical guide member 30 provided as described above guides the portion that is to be deformed in the pump member 20, so that such portion is collapsed straight in a uniform way.

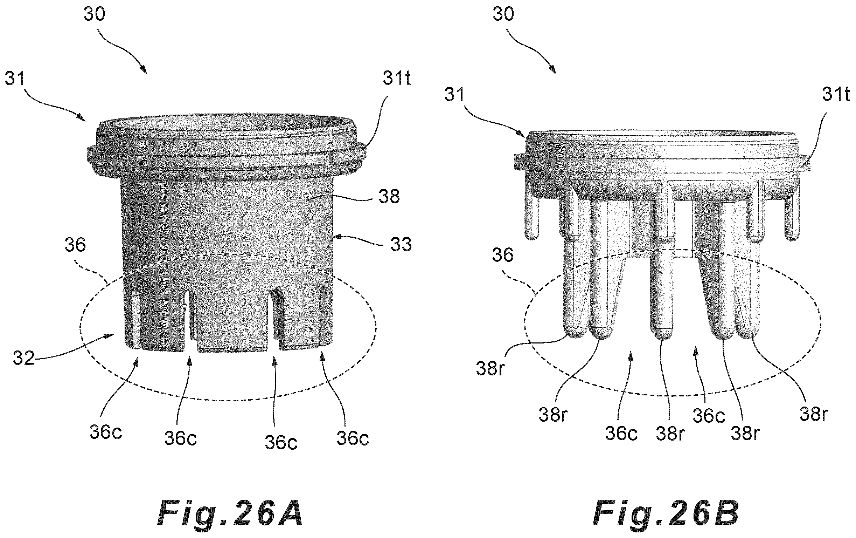

Slits may be formed in an extremity-side portion of the outer cylindrical portion 38. In the present embodiment, a plurality of extremity-side slits 36c are formed in at least part of an extremity-side cylindrical portion 36 of the cylindrical guide member 30 so as to be located along a circumferential direction (see Figure 26). It can be expected that the provision of such extremity-side slits 36c allows for the cylindrical guide member 30 to be reduced in weight or for its shape to be simplified. It is preferred for the extremity-side silts 36c to be provided at regular intervals in the circumferential direction (see Figure 26A). When such extremity-side slits 36c are provided, it is preferred for the total length of the widths (slit widths) of the extremity-side slits 36c in the circumferential direction to be equal to or less than a predetermined proportion of the entire circumferential length of the lower end portion 32 of the extremity-side cylindrical portion 36, e.g., 75% or less. This is based on the findings that even if the portion other than the extremity-side slits 36c constitutes a proportion of, for example about 25% or less, which causes the physical structure to be relatively weak, by arranging the extremity-side slits 36c uniformly along the circumferential direction of the extremity-side cylindrical portion 36, it is still possible for the guide member to sufficiently exercise its role of providing a trigger for guiding the portion that is to be deformed in the pump member 20 so as to be collapsed as intended. In addition, it is preferred for the plurality of extremity-side slits 36c to be arranged uniformly along the circumferential direction. Further, the extremity-side slits 36c may have other forms than the slit-like shape and they may be formed as, for example, spaces between a plurality of bracket-like rib portions 38r that are arranged in the circumferential direction (see Figure 26B).

Alternatively, a plurality of base end-side slits 35s may be provided in the base end-side cylindrical portion 35 of the cylindrical guide member 30 so as to be located along the circumferential direction (see Figures 24A-24D; in Figure 24A, the base end-side slits 35s are shown by imaginary lines). In this case, it is preferred for the sum of the widths of the base end-side slits 35s along the circumferential direction to be equal to or less than a predetermined proportion of the entire circumferential length of the upper end portion 31 of the cylindrical guide member 30, e.g., 50% or less. This is based on the findings that even if the portion other than the base end-side slits 35s constitutes a proportion of, for example about 50%, which causes the physical structure to be relatively weak, by arranging the base end-side slits 35s uniformly along the circumferential direction of the extremity-side cylindrical portion 36, it is still possible for the base end-side cylindrical portion 35 including the base end-side slits 35s to sufficiently exercise its function of being in contact with and thereby fixed to an inner peripheral surface 21i of the large diameter portion 21 of the pump member 20. In addition, it is preferred for the plurality of base end-side slits 35s to be arranged uniformly along the circumferential direction.

The cylindrical guide member 30 may have a structure in which a cylindrical portion 35 (which is referred to as the base end-side cylindrical portion 35 in the present specification, and can also be referred to as the "upper end cylindrical portion") in the upper end portion 31 of the cylindrical guide member 30 and a cylindrical portion (which is referred to as the extremity-side cylindrical portion 36 in the present specification, and can also be referred to as the "lower end cylindrical portion") are connected to each other (see Figure 23). In the cylindrical guide member 30 of the present embodiment, the outer diameter of the base end-side cylindrical portion 35 is larger than the outer diameter of the extremity-side cylindrical portion 36 (see Figure 23, etc.).

The cylindrical guide member 30 may also be formed such that the outer peripheral surface of the upper end portion 31 is in contact with and fixed to the inner peripheral surface 21i of the large diameter portion 21 of the pump member 20 (see Figure 22). In this case, an annular groove (not shown) is provided in the inner peripheral surface 21i of the large diameter portion 21 of the pump member 20, and an annular projected portion 31t that protrudes outward is provided on the outer peripheral surface of the upper end portion 31 of the cylindrical guide member 30 so as to engage with the groove (see Figure 22, etc.), and by engaging the annular projected portion 31t with the groove, the pump member 20 and the cylindrical guide member 30 can be positioned and fixed with respect to each other so as not to be easily disengageable.

### [Fourth Embodiment]

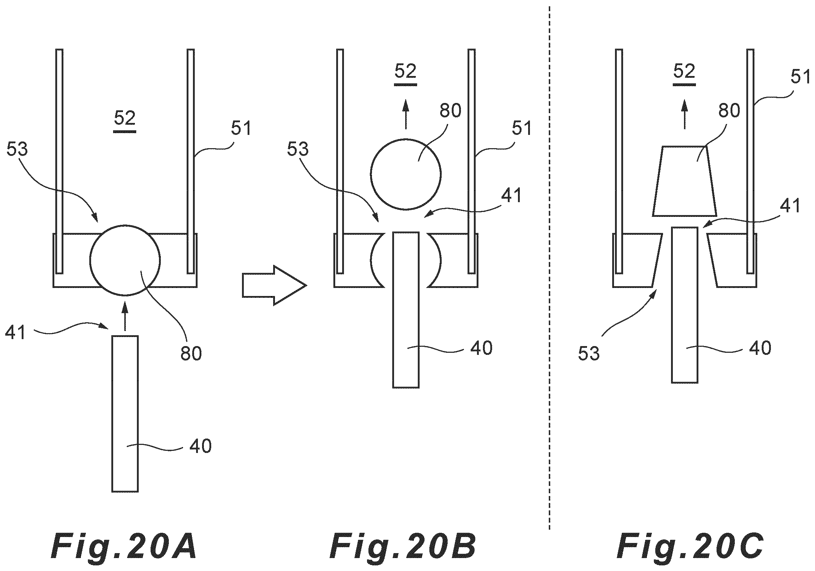

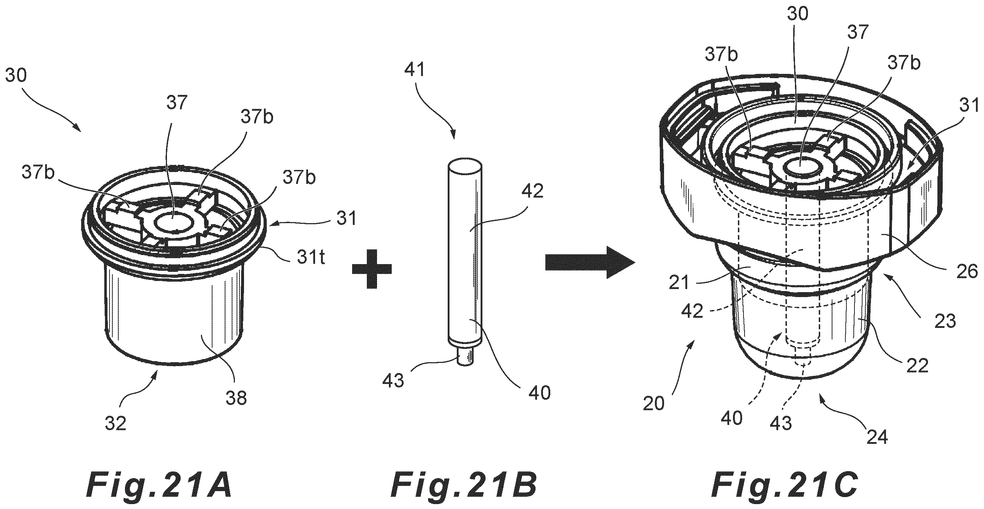

The embodiments described above each show the rod component 40 having the upper end portion 41 of a pointed shape, etc. The following description will describe, as the fourth embodiment of the present invention, an example in which the upper end portion 41 of the rod component 40 is formed as a flat surface (see Figures 20, 21, etc.).

The powdered medicine administration device 10 of the present embodiment is provided with the filling space which is filled with the powdered medicine M and a plug member 80 that seals the opening 53 of the filling space 52 (see Figure 20). The plug member 80 preferably has a shape that does not allow itself to easily pass through the opening 53 until it is pressed with an appropriate force, such as a spherical shape (see Figure 20A), a truncated conical shape (see Figure 20C), etc. The opening 53 is formed so as to fit the shape and the size of the plug member 80.

Although the rod component 40 of the powdered medicine administration device 10 of the present embodiment has an upper end portion 41 that is formed as a flat surface, this is merely an example. The upper end portion 41 of the rod component 40 may have any shape, as long as the plug member 80 can be pushed upward from below so as to render the opening 53 opened. As long as such configuration is achieved, the upper end portion 41 may have other shapes such as, for example, a concave shape that is entirely in contact with the surface of the spherical plug member 80.

Although the above-described embodiments are preferred examples of the present invention, the present invention is not limited thereto, and various modifications can be made thereto without departing from the gist of the present invention.

Although the powdered medicine administration device 10 described above is suitable as a device for administering a nasal medicine, the use thereof is not particularly limited thereto. For example, nasal administration is currently mainly used for topical treatment mainly for treating nasal inflammation. However, in recent times there have been a large number of commercialized nasal medicines that aim to absorb, through the nasal mucosa, medications that are expected to have systemic effects, such as medications for relieving migraine and cancer pain, and the use of the powdered medicine administration device 10 can be considered as including such application. Furthermore, studies have been conducted to transfer a medication from the olfactory region inside the nasal cavity to the brain, and the use of the powdered medicine administration device 10 also includes delivering the nasal medicine to the olfactory region inside the nasal cavity. In addition, in the development of medical products, the development of biomedicines that require strict dose control and strict storage management has been accelerating, and the need for nasal application of these medicines has been increasing. The use of the powdered medicine administration device 10 also includes such application.

All or part of the embodiments described may also be expressed as set forth in the appendices below. However, the present invention is not limited to the appendices set forth below.

### [Appendix 1]

A single-use type administration device for administering a predetermined dose of an object to be administered, such as a powdered medicine, into a nasal cavity, etc., the device comprising:
a pump member having a tapered shape as a whole, the pump member being deformed when pressed so as to reduce its internal volume; and
a cylindrical guide member that is arranged on an inner side of the pump member, the guide member guiding a portion that is to be deformed in the pump member.

### [Appendix 2]

The administration device according to Appendix 1, wherein the pump member is provided with a stepped portion at which the degree of tapering changes at a midpoint of the tapered shape.

### [Appendix 3]

The administration device according to Appendix 2, wherein the pump member has a shape that includes a tapered large diameter portion and a tapered small diameter portion which has a diameter smaller than the large diameter portion, and the stepped portion is formed between the large diameter portion and the small diameter portion.

### [Appendix 4]

The administration device according to any one of Appendices 1 to 3, wherein the pump member has a circular horizontal cross-sectional shape.

### [Appendix 5]

The administration device according to any one of Appendices 1 to 4, wherein a pressed portion which is to be pressed by a user is formed at an extremity portion of the pump member where an outer diameter reaches its minimum.

### [Appendix 6]

The administration device according to any one of Appendices 1 to 5, wherein the pump member has a shape that can be molded by injection molding.

### [Appendix 7]

The administration device according to Appendix 3, wherein the cylindrical guide member is arranged such that at least a portion thereof overlaps with the small diameter portion of the pump member.

### [Appendix 8]

The administration device according to Appendix 7, wherein an outer peripheral surface of the portion of the cylindrical guide member which overlaps with the small diameter portion of the pump member comes into contact with an inner peripheral surface of the small diameter portion.

### [Appendix 9]

The administration device according to Appendix 7 or 8, wherein a gap of 3 mm or less is formed between an outer peripheral surface of the portion of the cylindrical guide member which overlaps with the small diameter portion of the pump member and an inner peripheral surface of the small diameter portion.

### [Appendix 10]

The administration device according to any one of Appendices 7 to 9, wherein the cylindrical guide member has a structure in which: a base end-side cylindrical portion is provided at a base end portion located at an end in a direction along which the pump member is pressed; and an extremity-side cylindrical portion is provided at an extremity portion that is located at an end portion that faces the small diameter portion of the pump member, the extremity-side cylindrical portion extending continuously from the base end-side cylindrical portion.

### [Appendix 11]

The administration device according to Appendix 10, wherein an outer diameter of the base end-side cylindrical portion is larger than an outer diameter of the extremity-side cylindrical portion.

### [Appendix 12]

The administration device according to Appendix 3, wherein, in the cylindrical guide member, an outer peripheral surface of a base end portion located at an end in a direction along which the pump member is pressed is in contact with and fixed to an inner peripheral surface of the large diameter portion of the pump member.

### [Appendix 13]

The administration device according to Appendix 12, wherein a plurality of base end-side slits are provided at the base end portion of the cylindrical guide member, the base end-side slits being located along a circumferential direction.

### [Appendix 14]

The administration device according to Appendix 13, wherein a sum of widths of the base end-side slits along the circumferential direction of the cylindrical guide member is 50% or less of an entire circumferential length of the base end portion of the cylindrical guide member.

### [Appendix 15]

The administration device according to Appendix 5, wherein a rod-like guide member is provided on an inner circumferential side of the extremity portion of the pump member, the rod-like guide member suppressing a sideward movement of the extremity portion when the pump member is deformed.

### [Appendix 16]

The administration device according to Appendix 15, wherein a guide hole is provided at a central portion of the cylindrical guide member, the guide hole guiding the rod-like guide member so as to be slidable in a longitudinal direction thereof.

### [Appendix 17]

The administration device according to Appendix 16, wherein the rod-like guide member and the guide hole are provided along a central axis of the administration device.

### [Appendix 18]

The administration device according to Appendix 16 or 17, wherein the guide hole is formed on an inner side of a base end portion of the cylindrical guide member, the base end portion being located at an end in a direction along which the pump member is pressed.

### [Appendix 19]

The administration device according to Appendix 10 or 11, wherein a plurality of extremity-side slits are provided in at least part of the extremity-side cylindrical portion of the cylindrical guide member, the extremity-side slits being located along a circumferential direction.

### [Appendix 20]

The administration device according to Appendix 19, wherein a sum of widths of the extremity-side slits provided in at least part of the extremity-side cylindrical portion is 75% or less of an entire circumferential length of the extremity-side cylindrical portion.

### [Appendix 21]

The administration device according to any one of Appendices 15 to 17, wherein the rod-like guide member comprises a deformation suppressing portion that extends from a shaft thereof in a radial direction perpendicular to the shaft, the deformation suppressing portion suppressing a sideward movement of the pump member when being deformed.

### [Appendix 22]

The administration device according to Appendix 21, wherein the deformation suppressing portion is formed in a size and a shape in which at least part thereof comes into contact with an inner peripheral surface of the extremity-side cylindrical portion.

### [Appendix 23]

The administration device according to Appendix 21 or 22, wherein the deformation suppressing portion is formed in a shape in which the deformation suppressing portion extends in a wing-like shape from a shaft thereof in a radial direction perpendicular to the shaft.

### [Appendix 24]

The administration device according to any one of Appendices 15 to 17, wherein a tip end portion at a leading end of the rod-like guide member in a sliding direction has a planar shape, a projected shape, a recessed shape or a tapered shape.

### [Appendix 25]

The administration device according to any one of Appendices 15 to 17, further comprising: a nozzle member having a filling space that is filled with the object to be administered and a spray opening through which the object to be administered is sprayed; and a sealing member or a plug member that seals an opening of the filling space,
wherein the tip end portion at the leading end of the rod-like guide member is formed as a puncturing portion that punctures the sealing member or a plunger portion that pushes the plug member upward so as to provide an opening.

### [Appendix 26]

The administration device according to Appendix 25, wherein the pump member is connected to the opening of the filling space of the nozzle member, and air is sent out in response to a contracting motion of the pump member so as to cause the object to be administered to be sprayed through the spray opening of the nozzle member.

### Industrial Applicability

The present invention is suited to be applied to an administration device for various types of medicines such as medicines for nasal cavities, etc.

### Reference Signs List

- 10, 10': Powdered medicine administration device (administration device)
- 10C: Central axis
- 20, 20': Pump member
- 21: Large diameter portion
- 21i: Inner peripheral surface of the large diameter portion
- 22: Small diameter portion
- 22i: Inner peripheral surface of the small diameter portion
- 23: Narrowed portion (stepped portion)
- 24: Bottom portion (extremity portion)
- 24b: Boss portion
- 24d: Dish-like portion
- 25: Opening
- 26: Base portion
- 26c: Pump cover
- 29: Pressed portion
- 30: Cylindrical guide member
- (30C: Central portion)
- 31: Upper end portion (base end portion)
- 31t: Annular projected portion
- 32: Lower end portion of a guide member (a portion of the guide member which faces the extremity portion of the pump member)
- 33: Outer peripheral surface
- 34: Guide hole
- 34f: Locking portion
- 34i: Inner wall
- 35: Upper end cylindrical portion (base end-side cylindrical portion)
- 35p: Outer peripheral surface of the upper end cylindrical portion (base end-side cylindrical portion)
- 35s: Base end-side slit
- 36: Lower end cylindrical portion (extremity-side cylindrical portion)
- 36c: Extremity-side slit
- 37: Inner cylindrical portion
- 37e: End portion (of the inner cylindrical portion)
- 37b: Beam
- 38: Outer cylindrical portion (outside cylindrical portion)
- 38r: Rib portion
- 40, 40': Rod component (rod-like guide member)
- 41: Upper end portion
- 42: Shaft
- 42C: Groove
- 43: Lower end portion
- 44: Pointed piercer portion (puncturing portion)
- 44e: Rim portion
- 45: Wing-like portion (deformation suppressing portion)
- 45e: Outer peripheral edge of the wing-like portion
- 50: Nozzle member
- 51: Nozzle portion
- 52: Filling space
- 53: Opening (of the filling space)
- 54, 54': Spray opening
- 56, 57: Finger hook portion
- 60: Seal member
- 70: Tab (closing member)
- 80: Plug member
- M: Powdered medicine (object to be administered)
- S: Collapsing margin of the pump member

## Claims

1. A single-use type administration device for administering a predetermined dose of an object to be administered, such as a powdered medicine, into a nasal cavity, etc., the device comprising:
a pump member having a tapered shape as a whole, the pump member being deformed when pressed so as to reduce its internal volume; and
a cylindrical guide member that is arranged on an inner side of the pump member, the guide member guiding a portion that is to be deformed in the pump member.

2. The administration device according to claim 1, wherein the pump member is provided with a stepped portion at which the degree of tapering changes at a midpoint of the tapered shape.

3. The administration device according to claim 2, wherein the pump member has a shape that includes a tapered large diameter portion and a tapered small diameter portion which has a diameter smaller than the large diameter portion, and the stepped portion is formed between the large diameter portion and the small diameter portion.

4. The administration device according to claim 3, wherein the pump member has a circular horizontal cross-sectional shape.

5. The administration device according to claim 4, wherein a pressed portion which is to be pressed by a user is formed at an extremity portion of the pump member where an outer diameter reaches its minimum.

6. The administration device according to claim 3, wherein the pump member has a shape that can be molded by injection molding.

7. The administration device according to claim 3, wherein the cylindrical guide member is arranged such that at least a portion thereof overlaps with the small diameter portion of the pump member.

8. The administration device according to claim 7, wherein an outer peripheral surface of the portion of the cylindrical guide member which overlaps with the small diameter portion of the pump member comes into contact with an inner peripheral surface of the small diameter portion.

9. The administration device according to claim 7, wherein a gap of 3 mm or less is formed between an outer peripheral surface of the portion of the cylindrical guide member which overlaps with the small diameter portion of the pump member and an inner peripheral surface of the small diameter portion.

10. The administration device according to claim 7, wherein the cylindrical guide member has a structure in which: a base end-side cylindrical portion is provided at a base end portion located at an end in a direction along which the pump member is pressed; and an extremity-side cylindrical portion is provided at an extremity portion that is located at an end portion that faces the small diameter portion of the pump member, the extremity-side cylindrical portion extending continuously from the base end-side cylindrical portion.

11. The administration device according to claim 10, wherein an outer diameter of the base end-side cylindrical portion is larger than an outer diameter of the extremity-side cylindrical portion.

12. The administration device according to claim 4, wherein, in the cylindrical guide member, an outer peripheral surface of a base end portion located at an end in a direction along which the pump member is pressed is in contact with and fixed to an inner peripheral surface of the large diameter portion of the pump member.

13. The administration device according to claim 12, wherein a plurality of base end-side slits are provided at the base end portion of the cylindrical guide member, the base end-side slits being located along a circumferential direction.

14. The administration device according to claim 13, wherein a sum of widths of the base end-side slits along the circumferential direction of the cylindrical guide member is 50% or less of an entire circumferential length of the base end portion of the cylindrical guide member.

15. The administration device according to claim 5, wherein a rod-like guide member is provided on an inner circumferential side of the extremity portion of the pump member, the rod-like guide member suppressing a sideward movement of the extremity portion when the pump member is deformed.

16. The administration device according to claim 15, wherein a guide hole is provided at a central portion of the cylindrical guide member, the guide hole guiding the rod-like guide member so as to be slidable in a longitudinal direction thereof.

17. The administration device according to claim 16, wherein the rod-like guide member and the guide hole are provided along a central axis of the administration device.

18. The administration device according to claim 16, wherein the guide hole is formed on an inner side of a base end portion of the cylindrical guide member, the base end portion being located at an end in a direction along which the pump member is pressed.

19. The administration device according to claim 11, wherein a plurality of extremity-side slits are provided in at least part of the extremity-side cylindrical portion of the cylindrical guide member, the extremity-side slits being located along a circumferential direction.

20. The administration device according to claim 19, wherein a sum of widths of the extremity-side slits provided in at least part of the extremity-side cylindrical portion is 75% or less of an entire circumferential length of the extremity-side cylindrical portion.

21. The administration device according to claim 17, wherein the rod-like guide member comprises a deformation suppressing portion that extends from a shaft thereof in a radial direction perpendicular to the shaft, the deformation suppressing portion suppressing a sideward movement of the pump member when being deformed.

22. The administration device according to claim 21, wherein the deformation suppressing portion is formed in a size and a shape in which at least part thereof comes into contact with an inner peripheral surface of the extremity-side cylindrical portion.

23. The administration device according to claim 22, wherein the deformation suppressing portion is formed in a shape in which the deformation suppressing portion extends in a wing-like shape from a shaft thereof in a radial direction perpendicular to the shaft.

24. The administration device according to claim 16, wherein a tip end portion at a leading end of the rod-like guide member in a sliding direction has a planar shape, a projected shape, a recessed shape or a tapered shape.

25. The administration device according to claim 14, further comprising: a nozzle member having a filling space that is filled with the object to be administered and a spray opening through which the object to be administered is sprayed; and a sealing member or a plug member that seals an opening of the filling space,
wherein the tip end portion at the leading end of the rod-like guide member is formed as a puncturing portion that punctures the sealing member or a plunger portion that pushes the plug member upward so as to provide an opening.

26. The administration device according to claim 25, wherein the pump member is connected to the opening of the filling space of the nozzle member, and air is sent out in response to a contracting motion of the pump member so as to cause the object to be administered to be sprayed through the spray opening of the nozzle member.
